# EUROPEAN PATENT APPLICATION

(11) **EP 2 894 154 A1**
(43) Date of publication of application: **15.07.2015**
(21) Application number: 14151147.7
(22) Date of filing: 14.01.2014
(51) Int. Cl.: C07D 295/096, A61K 31/495, A61P 25/24

(54) **Synthesis of vortioxetine via (2-(piperazine-1-yl)phenyl)aniline intermediates**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention provides a new synthetic process for the production of 1-(2-((2,4-dimethylphenyl)thio)phenyl)piperazine (vortioxetine), a drug for the treatment of depression and anxiety, which is conducted via (2-(piperazine-1-yl)phenyl)aniline intermediates.

## Description

### Field of the Invention

This invention relates to a new and advantageous process for the synthesis of 1-(2-((2,4-dimethylphenyl)thio)phenyl)piperazine (vortioxetine) a drug for the treatment of depression and anxiety.

### Background of the Invention

Vortioxetine is disclosed as Example 1e in WO 2003/029232 A1 and is described as being prepared analogously to Example 1. The process used to prepare Example 1 involves the preparation of 1-(2-((2-(trifluoromethyl)phenyl)thio)phenyl)piperazine on a solid polystyrene support, followed by decomplexation using visible light irradiation, and purification by preparative LC-MS and ion-exchange chromatography. The overall yield for the preparation of vortioxetine is described as 17%.

Several alternative palladium catalyzed processes for the preparation of vortioxetine are described in Examples 17 to 25 of WO 2007/144005 A1. These processes describe the preparation of vortioxetine from 2,4-dimethylthiophenol and 2-bromoiodobenzene (or 1,2-dibromobenzene) starting materials *via* a 1-(2-bromo-phenylsulfanyl)-2,4-dimethyl-benzene intermediate. Each of these processes involves the use of a palladium catalyst and a phosphine ligand.

The preparation of vortioxetine is also described by Bang-Andersen et al. in J. Med. Chem. (2011), Vol. 54, 3206-3221. Here, in a first step, *tert*-butyl 4-(2-bromophenyl)piperazine-1-carboxylate intermediate is prepared from Boc-piperazine and 2-bromoiodobenzene in a palladium catalyzed coupling reaction. *tert*-Butyl 4-(2-bromophenyl)piperazine-1-carboxylate is then reacted with 2,4-dimethylthiophenol, again in the presence of palladium catalyst and a phosphine ligand, to provide Boc-protected vortioxetine. In the final step, vortioxetine is deprotected using hydrochloric acid to give vortioxetine hydrochloride.

WO 2013/102573 A1 describes a reaction between 1-halogen-2,4-dimethyl-phenyl, 2-halogen-thiophenol and an optionally protected piperazine in the presence of a base and a palladium catalyst consisting of a palladium source and a phosphine ligand.

Each of the above processes has disadvantages. The process described in WO 2003/029232 is low yielding and unsuitable for the large scale production of vortioxetine, whereas the processes described in WO 2007/144005 A1, WO 2013/102573 A1 and by Bang-Andersen *et al.* require the use of expensive starting materials, palladium catalyst and phosphine ligand. In addition, the toxicity of palladium is well known, Liu et al. Toxicity of Palladium, Toxicology Letters, 4 (1979) 469-473, and the European Medicines Agency' s Guideline on the Specification for Residues of Metal Catalysts sets clear limits on the permitted daily exposure to palladium arising from palladium residue within drug substances, www.ema.europa.eu. Thus it would be desirable to avoid the use of a palladium catalyst in the synthesis of vortioxetine and the subsequent purification steps required to remove palladium residue from the final pharmaceutical product.

### Summary of the Invention

Various aspects, advantageous features and preferred embodiments of the present invention as summarized in the following items, respectively alone or in combination, contribute to solving the object of the invention.
(1) A process for the preparation of a compound of formula **VIIa,** or a salt thereof, 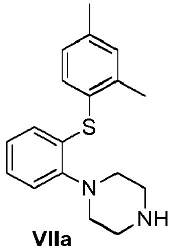 said process comprising the steps of
   a) reacting a compound of formula **IV,** or a salt thereof 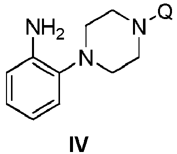 wherein Q represents hydrogen or an amino protecting group, with nitrite, and
   b) reacting the obtained diazo compound of formula **V** 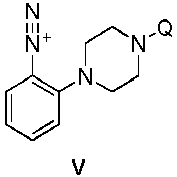 with a compound of formula **VI** 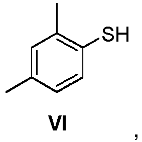
   c) optionally, converting the obtained compound of formula **VIIa** into the salt thereof.
   The term "amino protecting group" as employed herein means any group that is used for protection of amines. Typically, such group is selected from the group consisting of *tert-*butyloxycarbonyl (Boc), benzyloxycarbonyl (Cbz), fluorenylmethoxycarbonyl (Fmoc), unsubstituted or substituted benzyl, or benzenesulfonyl (Bs), p-toluenesulfonyl (Ts), 2-naphthylsulfonyl, trimethylsilyl (TMS), trifluoroacetyl (TFA), trityl (Tr), trichloroacetyl (TCA), formyl (CHO), acetyl (Ac), benzoyl (Bz), C₄-C₅-*tert*-alkyl, preferably *tert*-butyl (*t*-Bu). Preferably, the amino protecting group is *tert*-butoxycarbonyl (Boc) or acetyl (Ac).
(2) The process according to item 1, wherein the compound of formula **V** is not isolated.
(3) The process according to item 1, wherein Q in the compound of formula **IV** represents an amino protecting group, and step c) is followed by deprotecting the protected amino group to obtain the compound of formula **VIIa.**
(4) The process according to item 3, wherein neither the compound of formula **V** nor the amino protected intermediate is isolated.
(5) The process according to item 3, wherein the deprotection step is performed by using an acid selected from the groups consisting of HCl, HBr, H₂SO₄ and H₃PO₄ in a C₁-C₆ alcohol, preferably methanol or ethanol.
(6) The process according to item 1, wherein in step a) the compound of formula **IV** is dissolved in an acidic medium before reacting with the nitrite.
   The term "acidic medium" as employed herein means an acid containing medium wherein pH value in the presence of water is below 5.
(7) The process according to item 6, wherein the acidic medium is acetic acid.
(8) The process according to item 1, wherein the nitrite is sodium nitrite.
(9) The process according to item 1, wherein step a) is conducted at a temperature of from 0°C to 10°C.
(10) The process according to item 1, wherein in step b) a copper catalyst is present.
(11) The process according to item 1, wherein the compound of formula **IV** is prepared by a process comprising the steps of
   i) reacting a compound of formula I 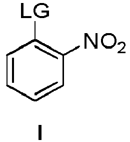 wherein LG represents a leaving group,
      with a compound of formula **II** 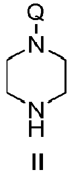 wherein Q is defined as in item 1,
      to obtain a compound of formula **III** 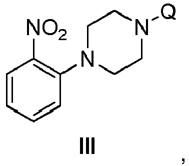 and
   ii) reducing the nitro group of the compound of formula **III,** to obtain the compound of formula **IV.**
   The term "leaving group" as employed herein means a molecular fragment that can be substituted by a nucleophile. The leaving species is able to stabilize the additional electron density that results from bond heterolysis. The preferred leaving groups are halogens, and sulfonates, such as tosylate.
(12) The process according to item 11, wherein the compound of formula **III** is not isolated.
(13) The process according to item 11 or item 12, wherein the leaving group is halogeno.
(14) The process according to item 13, wherein the leaving group is chloro.
(15) The process according to items 11 and 14, wherein step i) is conducted in an alcohol/water mixture.
(16) The process according to item 15, wherein the alcohol is a C₁-C₆ alcohol, preferably methanol or ethanol.
(17) The process according to item 16, wherein the reaction temperature of step i) is in a range from 0°C to 150°C, preferably the reaction temperature is about 70°C.
   The term "about" means approximately, in the region of, roughly, or around. When the term is used in conjunction with a numerical range, it modifies that range by extending the boundaries above and below the numerical values set forth. In general, the term "about" is used herein to modify a numerical value above and below the stated value by a variance of 10%.
(18) The process according to item 13, wherein the leaving group is fluorine.
(19) The process according to items 11 and 18, wherein step a) is conducted in a polar aprotic solvent, preferably DMSO or DMF.
   The term "polar aprotic solvent" as used herein means a polar solvent incapable of acting as a proton donor, for example, a solvent selected from the group consisting of tetrahydrofurane (THF), ethylacetate, acetone, dimethylformamide (DMF), acetonitrile and dimethyl sulfoxide (DMSO). Preferably, DMSO or DMF are used.
(20) The process according to item 19, wherein the reaction temperature of step i) is in a range from 0°C to 100°C, preferably the reaction temperature is about 50°C.
(21) The process according to any of the items 11 to 20, wherein in step i) a base is present, said base preferably being Na₂CO₃ or K₂CO₃.
   The term "base" as employed herein means a proton acceptor, preferably a water soluble proton acceptor or an alcoholate salt such as an alkali metal *tert*-butanolate salt, more preferably the proton acceptor is selected from the group consisting of carbonate salts, and hydroxides of alkaline or earth alkaline metals. The preferred bases are Na₂CO₃ and K₂CO₃.
(22) The process according to item 11, wherein in step ii) low valent reducing metal agents, such as iron, zinc or tin compounds, or catalytic hydrogenation are used.
(23) The process according to item 11, wherein step ii) is conducted in a basic medium in the presence of a compound selected from the group consisting of sodium dithionite (Na₂S₂O₄), thiourea dioxide (formamidinesulfinic acid) and sodium hydroxy-methanesulfinate dehydrate (Rongalite).
(24) The process according to item 1 or item 11, wherein Q represents an amino protecting group Boc or Ac.
(25) The process according to any one of the previous items, wherein the salt of the compound of formula **VIIa** is a hydrobromide salt or a hydrochloride salt.
(26) A compound of formula **VIIc** 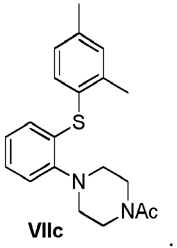
(27) Use of the compound as defined in item 26 in the preparation of vortioxetine or a salt thereof.
(28) A process for the preparation of a pharmaceutical composition comprising a compound of formula **VIIa,** or a salt thereof 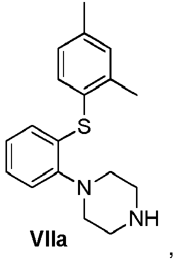 comprising the steps of:
   x) preparing a compound of formula **VIIa,** or a salt thereof, by carrying out a process according to any one of items 1 to 25, and
   y) mixing the compound of formula **VIIa,** or a salt thereof, with a pharmaceutically acceptable carrier and/or excipient.

The new process defined above provides vortioxetine, or salts thereof, in a high yield without the need of using the homogeneous catalysis with palladium catalysts and phosphine ligands, nor expensive starting materials, which provides an industrially applicable, environmentally friendly and economically improved process for obtaining vortioxetine, or salts thereof. The present invention further provides a new intermediate compound that is highly useful as a key intermediate in the preparation of vortioxetine or salts thereof. The intermediate is solid, crystalline, suitable for optional purification by precipitation or preferably recrystallization.

### Detailed Description of the Invention

The invention is described below in further detail by embodiments, without being limited thereto.

A general concept of the process of the present invention may be represented in Scheme 1.

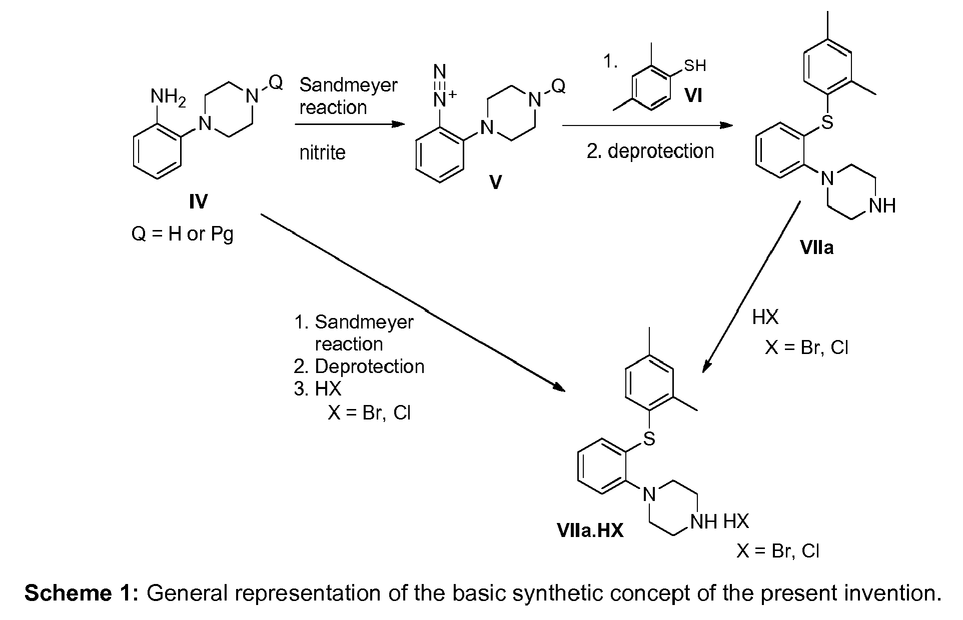

In the first step, the compound of formula **IV** is transformed to a diazonium compound of formula V. This can be achieved by reacting the compound of formula **IV** with an organic nitrite, for example, iso-amyl nitrite. Alternatively the compound of formula **IV** is dissolved in an acidic medium, for example in a solution of hydrohalic acid, for example, HCl or HBr, or in a solution of C₁-C₄-alkanoic acid, for example, acetic acid, which is followed by addition of an inorganic metal nitrite compound, for example, NaNO₂. The reaction is conducted in a temperature range from 0°C to 10°C. The obtained diazo intermediate compound of formula **V** is then reacted with 2,4-dimethylbenzenethiol **(VI),** optionally in the presence of a base. Finally, the compound of formula **VIIa** is obtained in a temperature range of from 60°C to 100°C, preferably at about 80°C. Optionally, a copper catalyst, for example, copper (II) salt, can be added to increase the reaction speed of the Sandmeyer reaction.

In case Q represents an amino protecting group (Pg), a deprotection is needed in the last step in order to afford vortioxetine **(VIIa).** This step may be conducted, for example, by using an acid selected from the groups consisting of HCl, HBr, H₂SO₄ and H₃PO₄ in a C₁-C₆ alcohol, preferably methanol or ethanol.

Preferably, the Sandmeyer reaction and, in case Q represents an amino protecting group, also the deprotection step may be performed as a one-pot process.

Optionally, the vortioxetine compound obtained by the process of the present invention can also be converted to a salt thereof, for example to a hydrobromide salt **(VIIa.HBr)** or a hydrochloride salt **(VIIa.HCl).**

In a particular embodiment, the compound of formula **IV** may be prepared by a reaction illustrated in Scheme 2.

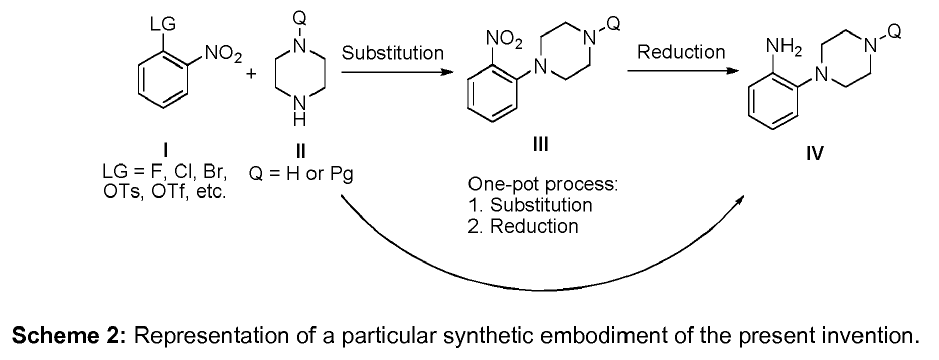

In the first step, the piperazine **(II)** is N-arylated with the compound of formula **I** to give the intermediate 1-(2-nitrophenyl)piperazine **(III).** In case Q is hydrogen, the compound of formula **III**can be isolated as a free amine or a salt. The nitro group in **III** is then reduced to an amino group to give intermediate 2-(piperazin-1-yl)aniline **(IV),** which, in case Q is hydrogen, can also be isolated as a free amine or a salt thereof. The reduction can be conducted with any method known to a skilled person. For example, catalytic hydrogenation can be applied, or, alternatively, a compound such as sodium dithionite (Na₂S₂O₄), thiourea dioxide (formamidinesulfinic acid) or sodium hydroxy-methanesulfinate dehydrate (Rongalite) can be used.

In a preferred embodiment, both substitution and reduction are conducted as a one-pot process, which consequently means that the compound of formula **IV** is obtained without isolation of **III**.

In a specific embodiment presented in Scheme 3, the 1-chloro-2-nitrobenzene **(Ia)** reacts with piperazine **(IIa)** in an alcohol/water mixture, preferably C₁-C₆ alcohols are used, most preferably MeOH or EtOH are used, at a temperature in a range from 0°C to 150°C, preferably at about 70°C, to give the intermediate **IIIa.** In this reaction piperazine **(IIa)** itself can act as a base or a compatible base, for example, Na₂CO₃ or K₂CO_{3,} can be used. Compound **IIIa** may be isolated as a free base or a salt thereof, preferably HCl salt.

In the next step, the nitro group of intermediate **IIIa** is reduced to the amino group according to standard reaction conditions known from the literature. Since the compound **IVa** is very soluble in water, and therefore very difficult to extract from aqueous media, a possible method for the reduction is, for example, catalytic hydrogenation, using Pd, Ni or Pt as a catalyst. Due to the poor solubility of compound **IVa** in organic solvents, the solvents suitable for the reaction are, for example, alcohols, preferably C₁-C₆ alcohols, most preferably MeOH or EtOH. Preferably, the reaction temperature for this step is at about 25°C. The obtained intermediate compound **IVa** can be isolated as a free base or as a salt, preferably HCl salt.

In the next reaction step, the Sandmeyer reaction is applied transforming compound **IVa** to vortioxetine **(VIIa).** Compound **IVa** is dissolved in an acidic media, preferably acetic acid, in a temperature range from 0°C to 10°C, preferably at about 5°C. To the obtained solution NaNO₂ is added slowly. Dependent on the type of the acid used, different types of organic solvents are used, for example, water can be used together with acetic acid. The obtained diazo compound **Va** then reacts with 2,4-dimethylbenzenethiol **(VI)** at a temperature of about 5°C, to give intermediate, which decomposes to vortioxetine **(VIIa)** in a temperature range from 60°C to 100°C, preferably at about 80°C.

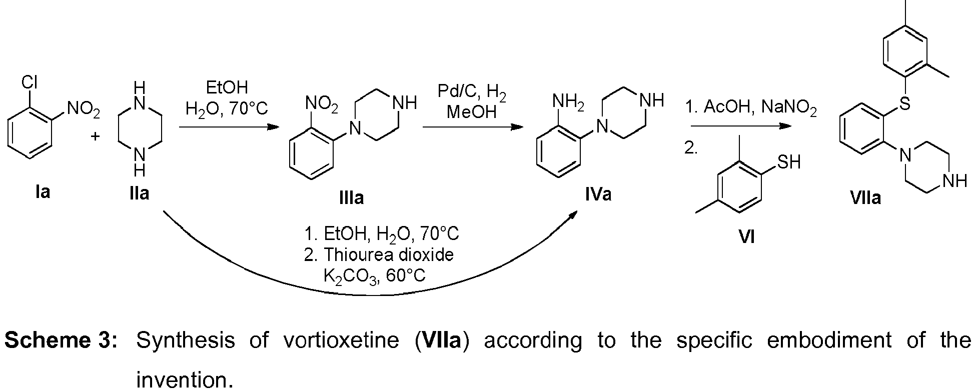

In a further specific embodiment of the present invention shown in Scheme 4, 1-fluoro-2-nitrobenzene **(Ib)** and N-Boc protected piperazine **(IIb)** are reacted to give the compound **IIIb.** This reaction occurs in polar aprotic solvents, preferably DMSO or DMF, or in polar protic solvents such as alcohols, preferably C₁-C₆ alcohols, most preferably in MeOH or EtOH. The reaction temperature depends on the solvent used. For example, if DMF is used then the reaction temperature should be about 50°C. Use of a base, for example, Na₂CO₃ or K₂CO₃, in the reaction towards **IIIb** is recommended but not obligatory. In case no additional base is used, two equivalents of N-Boc protected piperazine **(IIb)** may be required for the reaction to complete.

In the next step, the nitro group of intermediate **IIIb** is reduced to the amino group according to standard reaction conditions known from the literature. Apart from catalytic hydrogenation, using Pd, Ni or Pt as a catalyst, also other methods can be applied to obtain the compound of formula **IVb,** for example, sodium dithionite (Na₂S₂O₄) or thiourea dioxide (formamidinesulfinic acid) or sodium hydroxymethanesulfinate dehydrate (Rongalite) in basic media, can be used.

In the next reaction step, the Sandmeyer reaction is applied transforming compound **IVb** to N-Boc protected vortioxetine **(VIIb).** Compound **IVb** is dissolved in an acidic media, preferably aqueous solution of acetic acid, in a temperature range from 0°C to 10°C, preferably at about 5°C. In this case, only weak acids can be used, since Boc protection group is labile to strong acidic media. To the obtained solution NaNO₂ is added slowly. The obtained diazo compound **Vb** then reacts with 2,4-dimethylbenzenethiol **(VI)** at a temperature of about 5°C, to give intermediate, which decomposes to N-Boc protected vortioxetine **(VIIb)** in a temperature range from 60°C to 100°C, preferably at about 80°C.

Due to the presence of the N-Boc amino protecting group, the deprotection step is needed to obtain the compound of formula **VIIa.** For example an acid, preferably HCl, HBr, H₂SO₄ or H₃PO₄, in an alcohol, preferably C₁-C₆ alcohol, most preferably in MeOH or EtOH can be used. If for instance HCl is used for the deprotection step, the vortioxetine HCl salt **(VIIa.HCl)** is obtained. Similarly, if HBr is used, the vortioxetine HBr salt is obtained.

Last two steps (the Sandmeyer reaction and the deprotection step) can be compiled in a one-pot process using a strong acid such as HCl or HBr instead of AcOH.

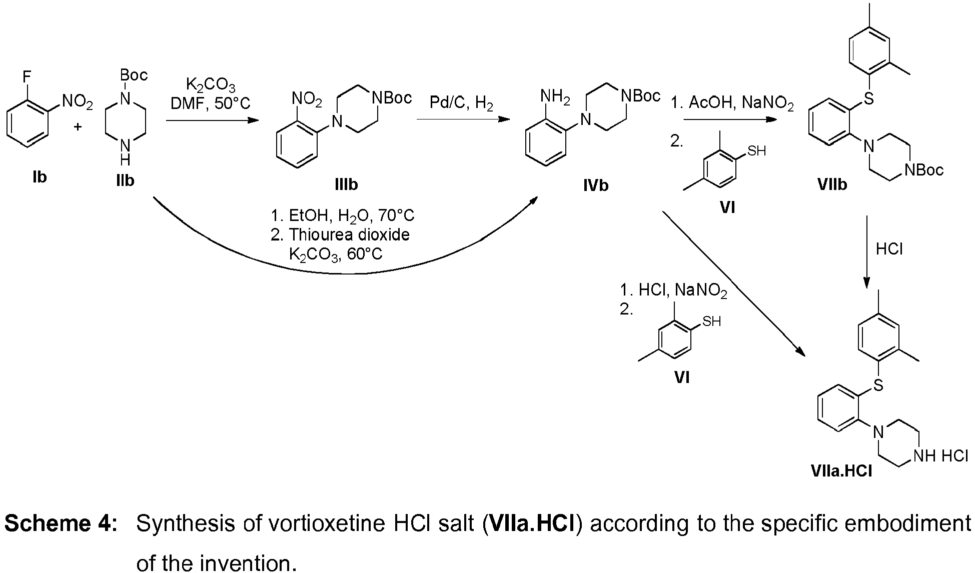

In a further specific embodiment of the present invention shown in Scheme 5, the 1-fluoro-2-nitrobenzene **(Ib)** and N-Ac protected piperazine **(IIc)** are reacted to give the compound of formula **IIIc,** which is then transformed to the compound **IVc** by nitro group reduction according to standard reaction conditions known from the literature. Apart from catalytic hydrogenation, using Pd, Ni or Pt as a catalyst, also other methods can be applied to obtain the compound of formula **IVc,** for example, sodium dithionite (Na₂S₂O₄) or thiourea dioxide (formamidinesulfinic acid) or sodium hydroxymethanesulfinate dehydrate (Rongalite) in basic media, can be used.

In the next reaction step, the Sandmeyer reaction is applied transforming compound **IVc** to N-Ac protected vortioxetine **(VIIc).** Compound **IVc** is dissolved in an acidic media, preferably aqueous solution of acetic acid, in a temperature range from 0°C to 10°C, preferably at about 5°C. Also in this case, only weak acids can be used, since Ac protection group is labile to strong acidic media. To the obtained solution NaNO₂ is added slowly. The obtained diazo compound **Vc** then reacts with 2,4-dimethylbenzenethiol **(VI)** at a temperature of about 5°C, to give intermediate, which decomposes to N-Ac protected vortioxetine **(VIIc)** in a temperature range from 60°C to 100°C, preferably at about 80°C.

Due to the presence of the N-Ac amino protecting group, the deprotection step is needed to obtain the compound of formula **VIIa.** For example an acid, preferably HCl, HBr, H₂SO₄ or H₃PO₄, in an alcohol, preferably C₁-C₆ alcohol, most preferably in MeOH or EtOH can be used. If for instance HCl is used for the deprotection step, the vortioxetine HCl salt **(VIIa.HCl)** is obtained. Similarly, if HBr is used, the vortioxetine HBr salt is obtained.

Last two steps (the Sandmeyer reaction and the deprotection step) can be compiled in a one-pot process using a strong acid such as HCl or HBr instead of AcOH.

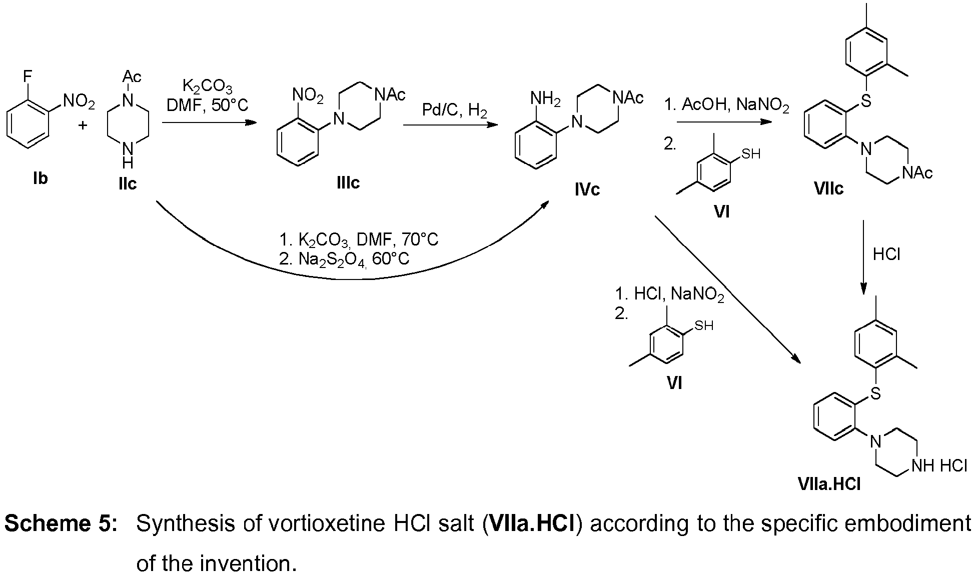

The vortioxetine compound, or a salt thereof, prepared according to the invention may be used or administered on its own, preferably it is administered as a pharmaceutical composition comprising vortioxetine, or a salt thereof, and a pharmaceutically acceptable excipient and/or carrier. Further, the vortioxetine compound, or a salt thereof, prepared according to the invention may be combined with other drugs, especially with drugs used for the treatment of depression and anxiety.

In a further aspect of the present invention, a pharmaceutical composition comprising the compound of formula **VIIa** (vortioxetine), or a salt thereof, is prepared by comprising the steps of preparing the compound of formula **VIIa,** or a salt thereof, as described above, and mixing the compound of formula **VIIa,** or a salt thereof, with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising vortioxetine, or a salt thereof, prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof, or particles containing the same may be coated by another substance. The powder mixture or particles containing the compound may also be dispensed into capsules.

The pharmaceutical composition comprising vortioxetine, or a salt thereof, prepared according to the invention in a desired dose is generally suitable to treat a disease or condition of a patient in need thereof, speciffically to display a desired activity when treating the depression and anxiety.

Further embodiment of the present invention resides in the provision of a valuable intermediate compound **VIIc** useful in the synthesis of a compound of formula **VIIa** (vortioxetine)

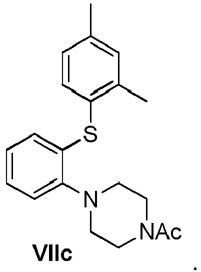

The following Examples are non-limiting and serve to illustrate the invention.

### Experimental Procedures

### Example 1: Preparation of 1-(2-nitrophenyl)piperazine (IIIa) from 1-chloro-2-nitrobenzene (Ia)

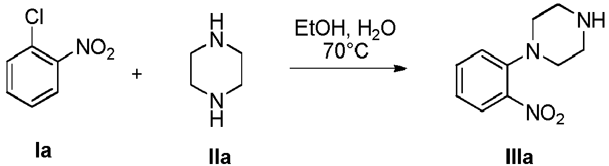

Mixture of **Ia** (11.0 g, 69.8 mmol), piperazine **(IIa)** (24.0 g, 279 mmol), EtOH (50 mL), and water (20 mL) was heated at 70°C for 40 h. EtOH was then evaporated under reduced pressure, water (80 mL) and 1 M NaOH (20 mL) were added, and product was extracted to EtOAc (3 x 50 mL). Combined organic layers were dried over Na₂SO₄, and solvent was evaporated to afford the title compound **IIIa** as red oil (14.1 g, 97% yield): ¹H NMR (CDCl₃, 500 MHz) *δ* 3.02 (m, 8H), 6.59 (br s, 1 H), 7.02 (m, 1 H), 7.13 (m, 1 H), 7.47 (m, 1 H), 7.74 (dd, *J* = 1.6, 8.1 Hz, 1 H); MS (ESI) m/z: 208 [MH]⁺.

### Example 2: Preparation of tert-butyl 4-(2-nitrophenyl)piperazine-1-carboxylate (IIIb) from 1-fluoro-2-nitrobenzene (Ib)

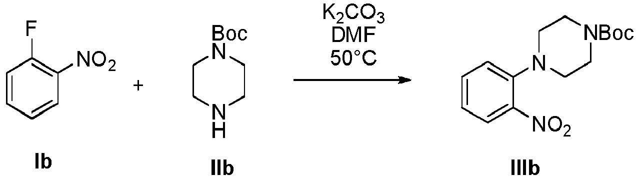

To a mixture of **IIb** (13.8 g, 74.4 mmol) and K₂CO₃ (11.7 g, 85.1 mmol) in DMF (60 mL) 1-fluoro-2-nitrobenzene **Ib** (10.0 g, 70.9 mmol) was added at 25°C. Obtained reaction mixture was stirred at 50°C for 17 h and then water (200 mL) was added. The product was extracted to toluene (3 x 100 mL), combined organic layers were dried over MgSO₄, and solvent was evaporated to afford the title compound **IIIb** as orange oil (21.7 g, 99% yield): ¹H NMR (CDCl₃, 500 MHz) *δ* 1.48 (s, 9H), 3.02 (m, 4H), 3.58 (m, 4H), 7.09 (m, 1 H), 7.15 (dd, *J* = 1.3, 8.3 Hz, 1 H), 7.50 (m, 1 H), 7.79 (dd, *J* = 1.6, 8.1 Hz, 1 H); MS (ESI) *m*/*z:* 308 [MH]⁺.

### Example 3: Preparation of 1-(4-(2-nitrophenyl)piperazin-1-yl)ethanone (IIIc) from 1-fluoro-2-nitrobenzene (Ib)

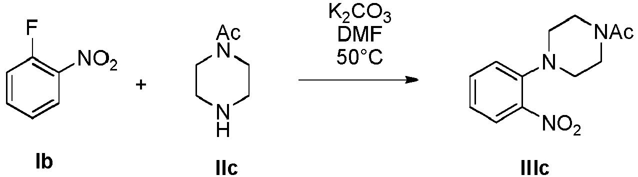

To a mixture of **IIc** (9.54 g, 74.4 mmol) and K₂CO₃ (11.7 g, 85.1 mmol) in DMF (60 mL) 1-fluoro-2-nitrobenzene **Ib** (10.0 g, 70.9 mmol) was added at 25°C. Obtained reaction mixture was stirred at 50°C for 17 h and then water (200 mL) was added. The product was extracted to EtOAc (3 x 150 mL), combined organic layers were washed with water (3 x 100 mL) and brine (2 x 100 mL), dried over MgSO₄, and solvent was evaporated to afford the title compound **IIIc** as orange oil which solidified upon standing. Orange crystals (17.4 g, 99% yield): ¹H NMR (CDCl₃, 500 MHz) *δ* 2.13 (s, 3H), 3.05 (m, 4H), 3.62 (m, 2H), 3.77 (m, 2H), 7.10-7.17 (m, 2H), 7.51 (m, 1 H), 7.80 (m, 1 H); MS (ESI) m/z: 250 [MH]⁺.

### Example 4: Preparation of 1-(2-nitrophenyl)piperazine hydrochloride (IIIa.HCl)

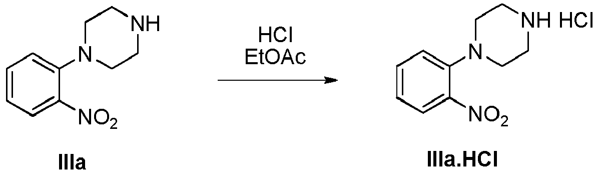

To a solution **IIIa** (13.2 g, 63.7 mmol) in EtOAc (100 mL) at room temperature 37% HCl (5 mL) was added. Obtained mixture was stirred at room temperature for 15 min, then precipitate was filtered off, washed with EtOAc (2 x 20 mL) and dried to afford 1-(2-nitrophenyl)piperazine hydrochloride **IIIa.HCl** as yellow powder (13.8 g, 86% yield): ¹H NMR (DMSO-*d*₆, 500 MHz) *δ* 3.15 (m, 4H), 3.24 (m, 4H), 7.22 (m, 1 H), 7.39 (m, 1 H), 7.64 (m, 1 H), 7.88 (dd, *J* = 1.5, 8.1 Hz, 1 H), 9.57 (br s, 2H).

### Example 5: Preparation of 2-(piperazin-1-yl)aniline hydrochloride (IVa.HCl)

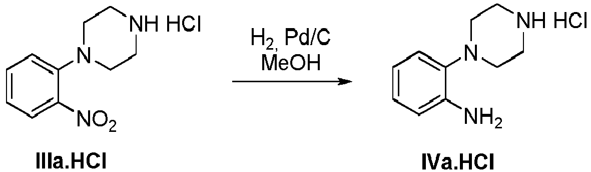

To a solution of **IIIa.HCl** (1.00 g, 4.10 mmol) in MeOH (10 mL) Pd/C (5%, 50 mg) is carefully added. Obtained mixture was hydrogenated under 10 bar H₂ for 17 h. Pd/C was then filtered off and solvent evaporated to afford an oil, which crystalized upon standing. EtOAc (5 mL) was then added, precipitate was filtered off and washed with EtOAc (2 x 5 mL) and dried to afford the title compound **IVa.HCl** as brown powder (0.82 g, 93% yield): ¹H NMR (D₂O, 500 MHz) *δ* 2.92 (m, 4H), 3.25 (m, 4H), 6.70-6.79 (m, 2H), 6.92 (m, 1 H), 7.00 (m, 1H).

### Example 6: Preparation of tert-butyl 4-(2-aminophenyl)piperazine-1-carboxylate (IVb)

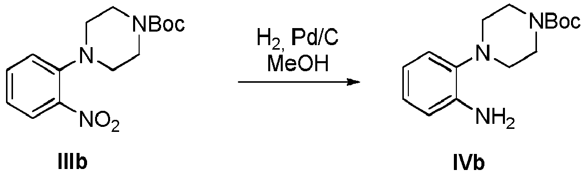

To a solution of **IIIb** (1.00 g, 3.25 mmol) in MeOH (10 mL) Pd/C (5%, 50 mg) was carefully added. Obtained mixture was hydrogenated under 10 bar H₂ for 14 h. Pd/C was then filtered off and solvent evaporated to afford an oil, which crystalized upon standing. The product was recrystallized from MeCy (10 mL), precipitate was filtered off, washed with MeCy (2 x 5 mL) and dried to afford the title compound **IVb** as light violet powder. (0.67 g, 74% yield): ¹H NMR (CDCl₃, 500 MHz) *δ* 1.50 (s, 9H), 2.87 (m, 4H), 3.57 (br s, 2H), 3.99 (m, 4H), 6.75 (m, 2H), 6.94-6.99 (m, 2H); MS (ESI) *m*/*z:* 278 [MH]⁺.

### Example 7: Preparation of tert-butyl 4-(2-aminophenyl)piperazine-1-carboxylate (IVb)

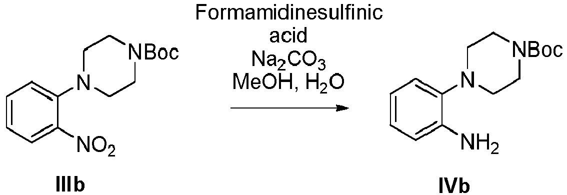

To a solution of **IIIb** (18.9 g, 61.6 mmol) in MeOH/water = 4:1 (190 mL) formamidinesulfinic acid (22.0 g, 203 mmol) and Na₂CO₃ (21.6 g, 203 mmol) were added. Obtained mixture was stirred at 50°C for 1.5 h. Salts were filtered off, and MeOH was evaporated under reduced pressure. Water (200 mL) was added to the remaining and product was extracted to EtOAc (150 mL). Organic layer was then washed with water (150 mL), dried over Na₂SO₄, and solvent was evaporated to afford the title compound **IVb** (8.25 g, 48% yield): ¹H NMR (CDCl₃, 500 MHz) *δ* 1.50 (s, 9H), 2.87 (m, 4H), 3.57 (br s, 2H), 3.99 (m, 4H), 6.75 (m, 2H), 6.94-6.99 (m, 2H); MS (ESI) *m*/*z:* 278 [MH]⁺.

### Example 8: Preparation of 1-(4-(2-aminophenyl)piperazin-1-yl)ethanone (IVc)

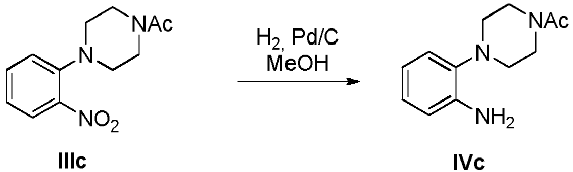

To a solution of **IIIc** (1.00 g, 4.01 mmol) in MeOH (10 mL) Pd/C (5%, 50 mg) was carefully added. Obtained mixture was hydrogenated under 5 bar H₂ for 18 h. Pd/C was then filtered off and solvent evaporated to afford brown cystals. MeCy (10 mL) was added, precipitate was filtered off, washed with MeCy (2 x 5 mL) and dried to afford the title compound **IVc** as light brown powder. (0.81 g, 92% yield): ¹H NMR (CDCl₃, 500 MHz) *δ* 2.16 (s, 3H), 2.91 (m, 4H), 3.61 (m, 2H), 3.76 (br s, 2H), 4.00 (m, 2H), 6.74-6.78 (m, 2H), 6.96-7.00 (m, 2H); MS (ESI) *m*/*z:* 220 [MH]⁺.

### Example 9: Preparation of 1-(2-((2,4-dimethylphenyl)thio)phenyl)piperazine (vortioxetine, VIIa)

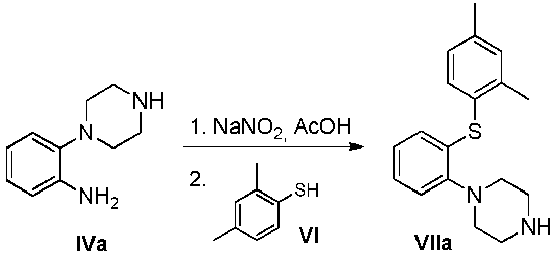

To a solution of **IVa** (0.35 g, 2.0 mmol) in AcOH (5 mL) and water (1 mL) at 5°C 48% NaNO₂ (0.14 g, 2.0 mmol) was slowly added. Obtained mixture was stirred at 5°C for 1.5 h, then **VI** was added (0.28 mL, 2.1 mmol). After stirring for 30 min at 5 °C, the reaction mixture was stirred at 70°C for 20 h. Volatile components were then evaporated under reduced pressure, 2 M NaOH (10 mL) was added, product was extracted to EtOAc (2x10 mL), combined organic layers were dried over Na₂SO₄, salts were filtered off and filtrate evaporated to afford crude product as dark red oil, which was then purified by chromatography (C18, NaH₂PO₄ buffer/MeCN) to give the title compound **VIIa** as yellow oil: ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1 H), 6.87 (m, 1 H), 7.04 (m, 1 H), 7.06-7.10 (m, 2H), 7.16 (m, 1 H), 7.39 (d, *J* = 7.8 Hz, 1 H); MS (ESI) *m*/*z:* 299 [MH]⁺.

### Example 10: Preparation of tert-butyl 4-(2-((2,4-dimethylphenyl)thio)phenyl)piperazine-1-carboxylate (VIIb)

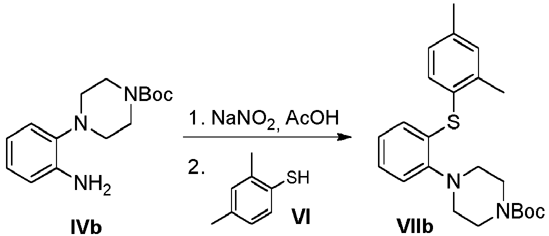

To a solution of **IVb** (0.53 g, 1.91 mmol) or a salt thereof **(IVb')** in AcOH (10 mL) at 2°C a solution of NaNO₂ (0.14 g, 2.0 mmol) in water (2 mL) was slowly added. After stirring at 2°C for 30 min to the reaction mixture was added **VI** (0.28 mL, 2.1 mmol), and the obtained reaction mixture was stirred at 20°C for 2 h. Water (30 mL) was added and the product was extracted to toluene (2 x 20 mL). Combined organic layers were dried over MgSO₄, salts were filtered off and filtrate was evaporated to afford crude product which was purified by chromatography (Silicagel, eluent MeCy/EtOAc mixture) to afford the title compound **VIIb** as yellow oil (0.32 g, 42% yield): ¹H NMR (CDCl₃, 500 MHz) *δ* 1.51 (s, 9H), 2.33 (s, 3H), 2.37 (s, 3H), 3.02 (m, 4H), 3.63 (m, 4H), 6.53 (dd, *J* = 1.4, 7.9 Hz, 1 H), 6.88 (m, 1 H), 7.02-7.05 (m, 2H), 7.08 (m, 1 H), 7.16 (m, 1 H), 7.38 (d, *J* = 7.8 Hz, 1 H); MS (ESI) m/z: 399 [MH]⁺.

### Example 11: Preparation of 1-(4-(2-((2,4-dimethylphenyl)thio)phenyl)piperazin-1-yl)ethanone (VIIc)

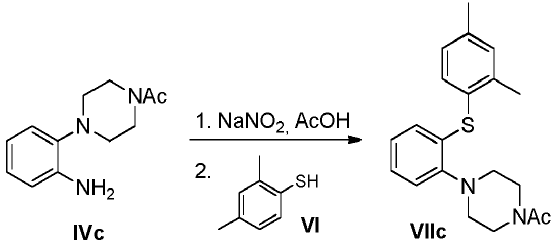

To a solution of **IVc** (0.50 g, 2.28 mmol) in AcOH (9 mL) and water (2 mL) 0°C NaNO₂ (165 mg, 2.39 mmol) was slowly added. Obtained mixture was stirred at 0°C for 30 min, then **VI** (0.34 mL, 2.51 mmol) was slowly added and obtained mixture was stirred at 5°C for 5 min and then at 70°C for 16 h. Solvent was then evaporated under reduced pressure, water (20 mL) and K₂CO₃ (2 g) were added and product was extracted to EtOAc (3 x 10 mL). Combined organic layers were dried over Na₂SO₄, salts were filtered off and filtrate evaporated to afford crude product which was purified by chromatography (MeCy/EtOAc) to afford the title compound **VIIc** as yellow oil which crystalized upon standing. Yellow crystals (0,34 g, 44% yield): ¹H NMR (CDCl₃, 500 MHz) *δ* 2.16 (s, 3H), 2.33 (s, 3H), 2.37 (s, 3H), 3.05 (m, 4H), 3.56 (m, 2H), 3.81 (m, 2H), 6.54 (dd, *J =* 1.4, 8.0 Hz, 1 H), 6.89 (m, 1 H), 7.03 (m, 2H), 7.09 (m, 1 H), 7.16 (m, 1 H), 7.37 (d, *J* = 7.8 Hz, 1 H); MS (ESI) *m*/*z:* 341 [MH]⁺.

### Example 12: Preparation of 1-(2-((2,4-dimethylphenyl)thio)phenyl)piperazine hydrobromide (vortioxetine HBr, VIIa.HBr)

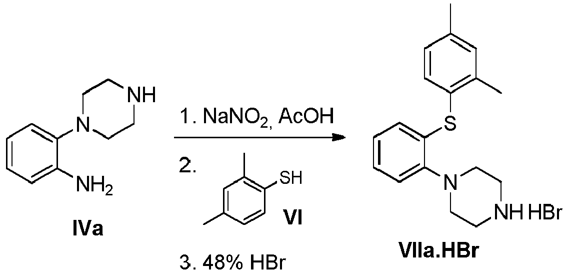

To an ice-cold (0°C) stirred solution of **IVa** (3.50 g, 20.0 mmol) or a salt thereof in AcOH (35 mL) and water (5 mL) a solution of NaNO₂ (1.38 g, 20.0 mmol) in water (5 mL) was added slowly. After stirring at 0°C for 1 h, to the reaction mixture was slowly added **VI** (2.98 mL, 22.0 mmol) and obtained reaction mixture was stirred at 5°C for 30 min, and at 60°C for 2 h. Solvents were evaporated under reduced pressure, water (75 mL) and Na₂CO₃ (10 g) were added. After extraction to EtOAc (2 x 50 mL) combined organic layers were dried over Na₂SO₄, salts were filtered off and filtrate was evaporated to afford crude product as brown syrup, which was then dissolved in EtOH (10 mL). Water (75 mL) was then added followed by 48% HBr (2.25 mL). After stirring at room temperature for 20 h precipitate was filtered off, washed with water (2 x 20 mL) and dried. Powder was then washed with EtOAc (2 x 20 mL) and dried again to afford the title compound **VIIa.HBr** as beige powder (3.82 g): ¹H NMR (DMSO-*d*₆, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, *J* = 7.8 Hz, 1 H), 8.85 (br, 2H).

### Example 13: Preparation of 1-(2-((2,4-dimethylphenyl)thio)phenyl)piperazine hydrochloride (vortioxetine HCl, VIIa.HCl)

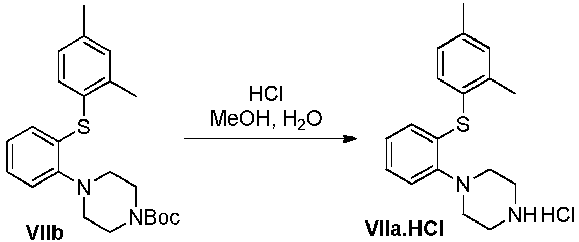

A mixture of **VIIb** (1.78 g, 4.47 mmol), MeOH (10 mL,) water (2 mL), and 37% HCl (6 mL) was stirred at room temperature for 3 days. To the reaction mixture water (30 mL) was added, precipitate was filtered off. After washing with water (10 mL) and MeOAc (2 x 20 mL) it was dried to afford title compound **VIIa.HCl.** Off white powder (1.27 g, 85% yield): DSC: Onset 230.31 °C, Peak 323.15°C.

### Example 14: Preparation of 1-(2-((2,4-dimethylphenyl)thio)phenyl)piperazine (vortioxetine, VIIa)

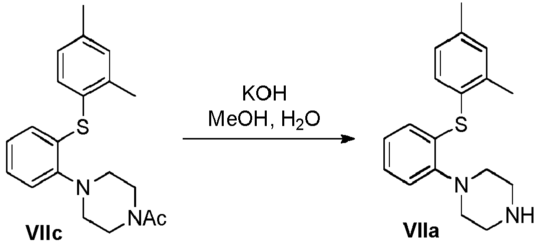

To a mixture of **VIIc** (0.50 g, 1.47 mmol) in MeOH (10 mL) and water (2.5 mL) at room temperature KOH (1.50 g) was added. Obtained mixture was stirred at reflux for 24 h, MeOH was then partly evaporated and water (20 mL) added. Precipitate was filtered off, washed with water (2 x 10 mL) and dried to afford the title compound **VIIa** as beige powder (0.44 g, total yield): ¹H NMR (CDCl₃, 500 MHz) *δ* 1.63 (br s, 1H), 2.33 (s, 3H), 2.37 (s, 3H), 3.02-3.09 (m, 8H), 6.52 (m, 1H), 6.87 (m, 1H), 7.04 (m, 1 H), 7.06-7.10 (m, 2H), 7.16 (m, 1 H), 7.39 (d, J = 7.8 Hz, 1 H); MS (ESI) m/z: 299 [MH]⁺.

### Example 15: Preparation of 1-(2-((2,4-dimethylphenyl)thio)phenyl)piperazine hydrobromide (vortioxetine HBr, VIIa.HBr)

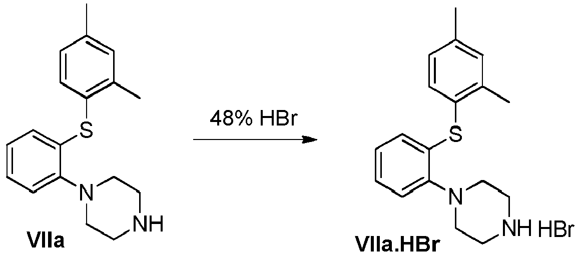

To a solution of vortioxetine **VIIa** (1.80 g, 6.03 mmol) in iPrOAc (20 mL) at room temperature 48% HBr (0.68 mL, 6.03 mmol) was slowly added. Obtained mixture was stirred at room temperature for 1 h, white precipitate was then filtered off, washed with acetone (2 x 20 mL), and dried to afford the title compound **VIIa.HBr** as white powder (2.15 g, 94% yield): DSC: Onset 221.74°C, Peak 223.86°C; ¹H NMR (DMSO-*d*₆, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, *J =* 7.8 Hz, 1 H), 8.85 (br, 2H).

## Claims

1. A process for the preparation of a compound of formula **VIIa,** or a salt thereof, 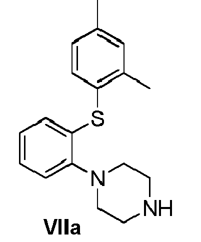 said process comprising the steps of
a) reacting a compound of formula **IV,** or a salt thereof 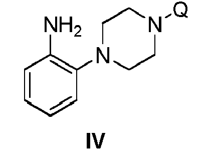 wherein Q represents hydrogen or an amino protecting group,
with nitrite, and
b) reacting the obtained diazo compound of formula **V** 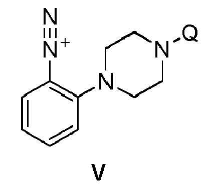 with a compound of formula **VI** 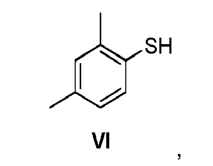
c) optionally, converting the obtained compound of formula **VIIa** into the salt thereof.

2. The process according to claim 1, wherein the compound of formula V is not isolated.

3. The process according to claim 1, wherein Q in the compound of formula **IV** represents an amino protecting group, and step c) is followed by deprotecting the protected amino group to obtain the compound of formula **VIIa.**

4. The process according to claim 1, wherein in step a) the compound of formula **IV** is dissolved in an acidic medium.

5. The process according to claim 4, wherein the acidic medium is acetic acid.

6. The process according to claim 1, wherein the nitrite is sodium nitrite.

7. The process according to claim 1, wherein step a) is conducted at a temperature of from 0°C to 10°C.

8. The process according to claim 1, wherein the compound of formula **IV** is prepared by a process comprising the steps of
i) reacting a compound of formula I 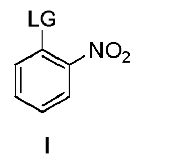 wherein LG represents a leaving group,
with a compound of formula **II** 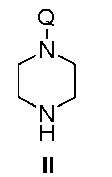 wherein Q is defined as in claim 1,
to obtain a compound of formula **III,** or a salt thereof 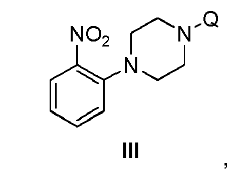 and
ii) reducing the nitro group of the compound of formula **III,** or a salt thereof, to obtain the compound of formula **IV,** or a salt thereof.

9. The process according to claim 8, wherein the compound of formula **III** is not isolated.

10. The process according to claim 8 or claim 9, wherein the leaving group is halogen.

11. The process according to claim 1 or claim 8, wherein Q represents an amino protecting group Boc or Ac.

12. The process according to any one of the previous claims, wherein the salt of the compound of formula **VIIa** is a hydrobromide salt or a hydrochloride salt.

13. A compound of formula **VIIc**

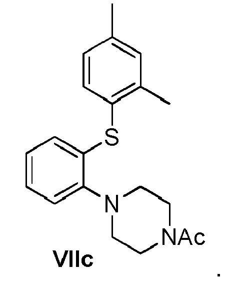

14. Use of the compound as defined in claim 13 in the preparation of vortioxetine or a salt thereof.

15. A process for the preparation of a pharmaceutical composition comprising a compound of formula **VIIa,** or a salt thereof 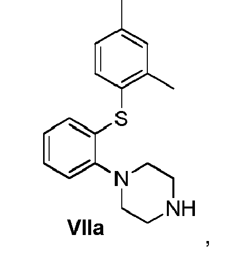 comprising the steps of:
x) preparing a compound of formula **VIIa,** or a salt thereof, by carrying out a process according to any one of claims 1 to 12, and
y) mixing the compound of formula **VIIa,** or a salt thereof, with a pharmaceutically acceptable carrier and/or excipient.
